# EUROPEAN PATENT APPLICATION

(11) **EP 1 160 321 A1**
(43) Date of publication of application: **05.12.2001**
(21) Application number: 00401531.9
(22) Date of filing: 31.05.2000
(51) Int. Cl.: C12N 15/12, C07K 14/47, C07K 16/18, A61K 38/17

(54) **Kidney Injury Novel Gene-1: Isolation and therapeutic applications**

(71) Applicant: SANOFI-SYNTHELABO, 75013 Paris (FR)
(72) Inventor: Bidouard, Jean-Pierre, 91380 Chilly Mazarin (FR); Culouscou, Jean-Michel, 92500 Rueil Malmaison (FR); Faure, Cécile, 92500 Rueil Malmaison (FR); Gouhier, Caroline, 1005 Lausanne (CH); Janiak, Philip, 91190 Gif-sur-Yvette (FR)
(74) Representative: Ludwig, Jacques

(57) **Abstract**

KING-1 (Kidney Injury Novel Gene-1) polypeptides and polynucleotides and methods for producing such polypeptides by recombinant techniques are disclosed. Also disclosed are methods for utilizing KING-1 polypeptides and polynucleotides in the design of protocols for the treatment of several pathologies, and diagnostic assays for such conditions.

## Description

The invention relates to newly identified polynucleotides, to polypeptides encoded by them and to the use of such polynucleotides and polypeptides. The invention also relates to their production. More particularly, the polynucleotides and polypeptides of the invention relate to a newly identified gene, hereinafter referred to as Kidney Injury Novel Gene-1 (KING-1). Two variants of human KING-1, as well as rat KING-1, are hereinafter disclosed. The invention also relates to inhibiting or activating the action of such polynucleotides and polypeptides and to their therapeutic applications.

Acute renal failure is a devastating multifactorial syndrome with high incidence and mortality. The pathophysiology of acute renal failure is complex. Acute ischemic injury to renal tubular epithelial cells leads to massive cell death due to necrosis and sloughing, resulting in tubular obstruction and dramatic lost of renal function. This injury process is followed by dedifferentiation, proliferation, then differentiation of survival cells, allowing reparative cellular processes and regeneration of damaged tubular epithelium, culminating in the restoration of renal function (Sutton T.A. and Molitoris B.A. (1998) Semin. Nephrol. 8:490-497; Thadhani R. et al. (1996) N. Engl. J. Med. 334:1448-1460; Toback F.G. (1992) Kidney Int. 41:226-246; Bonventre J.V. (1993) Kidney Int. 43:1160-1178). Dialysis continues to be the major supportive intervention in severe acute renal failure.

Identification and characterization of genes whose expression is induced following ischemia and reperfusion would allow a better understanding of the physiopathology that occurs in ischemic acute renal failure. Such genes might be involved in either the process of ischemic injury of renal tubular cells (deletorious effect) or the phase of restoration of renal function (benefic effect). Their characterization may be helpful for the development of new therapeutics.

In one aspect, the invention relates to KING-1 polypeptides and polynucleotides, and to recombinant materials and methods for their production. Another aspect of the invention relates to methods for using such KING-1 polypeptides and polynucleotides. Such uses include the treatment of ischemic renal failure, glomerulonephritis, chronic renal failure, diabetic nephropathy, cardiac ischemia, myocardial infarction, hypertension, congestive heart failure, inflammatory and proliferative diseases, among others.

In still another aspect, the invention relates to methods to identify KING-1 agonists and antagonists using the materials provided by the invention, and treatment conditions associated with the use of such compounds. Yet another aspect of the invention relates to diagnostic assays for detecting diseases associated with inappropriate KING-1 activity or levels.

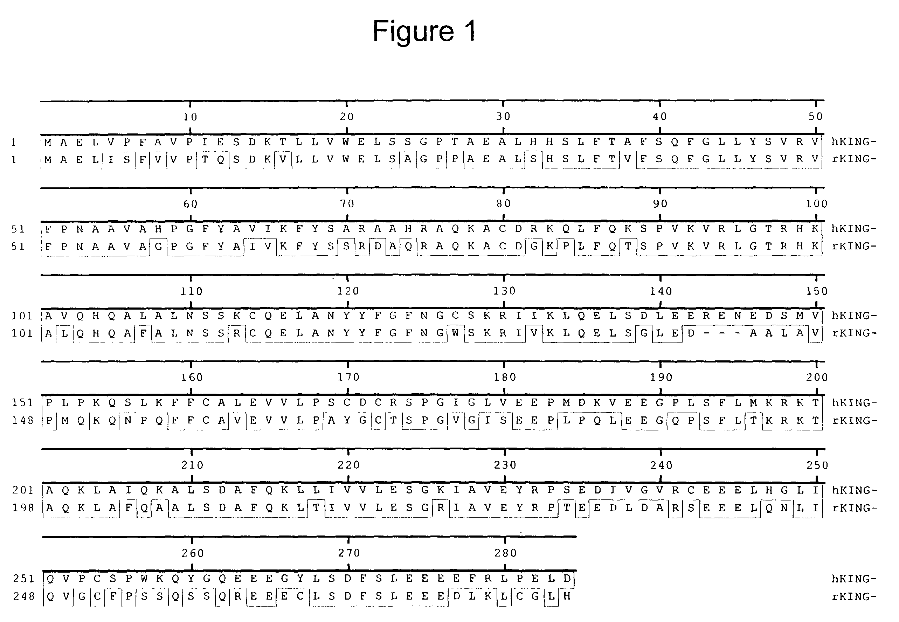

Figure 1 illustrates the alignment of human and rat KING-1 protein sequences using the Clustal algorithm of MEGALIGN (version 3.18) from LASERGENE.

The following definitions are provided to facilitate understanding of certain terms used frequently herein.

"KING-1" refers to a polypeptide comprising the amino acid sequence set forth in SEQ ID NO: 4, SEQ ID NO: 5 or SEQ ID NO: 6, or an allelic variant thereof, as well as other variants as described further.

"KING-1 activity" or "biological activity of KING-1" refers to the metabolic or physiologic function of said KING-1 including similar activities or improved activities or these activities with decreased undesirable side-effects. Also included are antigenic and immunogenic activities of said KING-1.

"KING-1 gene" or "KING-1 polynucleotide" refers to a polynucleotide comprising the nucleotide sequences set forth in SEQ ID NO: 1, SEQ ID NO: 2, SEQ ID NO: 3, or allelic variants thereof and/or their complements, as described in the following specification.

"Antibodies" as used herein include polyclonal and monoclonal antibodies, chimeric, single chain, and humanized antibodies (an antibody ― mouse or any other - with some specific parts changed to human homologous regions), as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein. For example, recombinant DNA molecules contained in a vector are considered isolated for the purposes of the invention. Further examples of isolated nucleic acid molecules include recombinant DNA molecules maintained in heterologous host cells or purified DNA molecules in solution. Isolated RNA molecules include *in vivo* or *in vitro* RNA transcripts of the DNA molecules of the invention. Isolated nucleic acid molecules according to the invention further include such molecules produced synthetically.

"Polynucleotide" generally refers to any polyribonucleotide or polydeoxribonucleotide, which may be unmodified RNA or DNA or modified RNA or DNA. "Polynucleotides" include, without limitation, single- and double-stranded DNA, DNA that is a mixture of single- and double-stranded regions, single- and double-stranded RNA, and RNA that is a mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNA or RNA containing one or more modified bases and DNA or RNA with backbones modified for stability or for other reasons as explained in the present application. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. Thus, "polynucleotide" also encompasses variants of the nucleic acid molecules of the invention which encode portions, analogs or derivatives of the KING-1 genes. Variants may occur naturally, such as natural allelic variants. By an "allelic variant" is intended one of several alternate forms of a gene occupying a given locus on a chromosome of an organism. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, Proteins - Structure and molecular properties, 2nd Ed., T. E. Creighton, W. H. Freeman and Company, New York, 1993 and Wold, F, Post-translational Protein Modifications: Perspectives and Prospects, p. 1-12 in Post-translational covalent modification of proteins, B. C. Johnson, Ed., Academic Press, New York, 1983; Seifter *et al.*, "Analysis for protein modifications and non-protein cofactors", *Meth*. *Enzymol.* (1990) 182:626-646 and Rattan *et al.*, "Protein Synthesis: Post-translational Modifications and Aging", *Ann*. *NY Acad*. *Sci.* (1992) 663:48-62.

"Variant" as the term is used herein, is a polynucleotide or polypeptide that differs from a reference polynucleotide or polypeptide respectively, but retains its essential properties. A typical variant of a polynucleotide differs in nucleotide sequence from another reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be naturally occurring, such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g. : Computational Molecular Biology, Lesk, A.M., ed., Oxford University Press, New York, 1988; Biocomputing Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991. While there exists a number of methods to measure identity between two polynucleotide or polypeptide sequences, the term "identity" is well known to skilled artisans (Carillo, H., and Lipton, D., *SIAM J*. *Applied Math.* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J. Applied Math.* (1988) 48:1073. Methods to determine identity and similarity are codified in computer programs. Preferred computer program methods to determine identity and similarity between two sequences include, but are not limited to, GCS program package (Devereux, J., *et al*., *Nucleic Acids Research* (1984) 12(1):387), BLASTP, BLASTN, FASTA (Atschul, S.F. *et al*., *J*. *Molec*. *Biol.* (1990) 215:403).

As an illustration, by a polynucleotide having a nucleotide sequence having at least, for example, 95% "identity" to a reference nucleotide sequence is intended a polynucleotide in which the nucleotide sequence is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence is intended a polypeptide having an amino acid sequence identical to the reference sequence except that the polypeptide sequence may include up to five amino acid alterations per each 100 amino acids of the reference amino acid. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

In one aspect, the invention relates to isolated KING-1 polypeptides. The KING-1 polypeptides include the polypeptide of SEQ ID NO: 4, SEQ ID NO: 5 or SEQ ID NO: 6; as well as polypeptides comprising the amino acid sequence of SEQ ID NO: 4, SEQ ID NO: 5 or SEQ ID NO: 6; and polypeptides comprising an amino acid sequence which has at least 80% identity to that of SEQ ID NO: 4, SEQ ID NO: 5 or SEQ ID NO: 6 over its entire length, and still more preferably at least 90% identity, and even still more preferably at least 95% identity to SEQ ID NO: 4, SEQ ID NO: 5 or SEQ ID NO: 6. Furthermore, those with at least 97-99% identity are highly preferred. Also included within KING-1 polypeptides are polypeptides having the amino acid sequence which have at least 80% identity to the polypeptide having the amino acid sequence of SEQ ID NO: 4, SEQ ID NO: 5 or SEQ ID NO: 6 over its entire length, and still more preferably at least 90% identity, and even still more preferably at least 95% identity to SEQ ID NO: 4, SEQ ID NO: 5 or SEQ ID NO: 6. Furthermore, those with at least 97-99% identity are highly preferred.

It will be recognized that some amino acids of the KING-1 polypeptides can be varied without significant effect on the structure or function of the protein. Guidance concerning with amino acid changes which are likely to be phenotypically silent can be found in Bowie, J. U. et al, "Deciphering the Message in Protein Sequences : Tolerance to Amino Acid Substitutions", *Science* 247 : 1306-1310 (1990).

Thus, the invention further provides variants of the KING-1 polypeptides which show substantial KING-1 polypeptide activity or which include regions of KING-1 protein such as the protein portions discussed below. Such mutants include KING-1 polypeptides in which deletions, insertions, type substitutions, inversions and/or repeats have occurred.

Thus, the fragment, derivative or analog of the polypeptide of SEQ ID NO: 4, SEQ ID NO: 5 or SEQ ID NO: 6, or that encoded by the deposited cDNA, may be :
- one in which one or more of the amino acid residues are substituted with a conserved or non-conserved amino acid residue, preferably a conserved amino acid residue, and such substituted amino acid residue may or may not be one encoded by the genetic code, or
- one in which one or more of the amino acid residues includes a substituent group, or
- one in which the mature polypeptide is fused with another compound, such as a compound for increasing the half-life of the polypeptide (for example, polyethylene glycol), or
- one in which additional amino acids are fused to the mature polypeptide, such as the IgG Fc fusion region peptide or leader or secretory sequence which is employed for purification of the mature polypeptide or a pro-protein sequence. Such fragments, derivatives and analogs are deemed to be within the scope of the invention.

Of particular interest are substitutions of charged amino acids with another charged amino acid and with neutral or negatively charged amino acids. The latter results in proteins with reduced positive charge to improve the characteristics of the KING-1 protein, for example for prevention of aggregation.

As indicated, changes are preferably of a minor nature, such as conservative amino acid substitutions that do not significantly affect the activity of the protein, such as those indicated hereafter.

| | |
|---|---|
| Aromatic | Phenylalanine |
| | Tryptophan |
| | Tyrosine |
| Hydrophobic | Leucine |
| | Isoleucine |
| | Valine |
| Polar | Glutamine |
| | Asparagine |
| Basic | Arginine |
| | Lysine |
| | Histidine |
| Acidic | Aspartic acid |
| | Glutamic acid |
| Small | Alanine |
| | Serine |
| | Threonine |
| | Methionine |
| | Glysine |

Generally, the number of amino acid substitutions will not be more than 50.

Amino acids in the KING-1 polypeptide of the invention that are essential for function can be identified by methods known in the art, such as site-directed mutagenesis or alanine-scanning mutagenesis (Cunnigham and Wells, *Science* 244 : 1081-1085 (1989)). The latter procedure introduces single alanine mutations at every residue in the molecule. The resulting mutant molecules are then tested for biological activity such as receptor binding or *in vitro* proliferative activity. Sites that are critical for ligand-receptor binding can also be determined by structural analysis, e.g. crystallization, nuclear magnetic resonance or photoaffinity labeling (Smith et al., *J. Mol*. *Biol.* 224 : 899-904 (1992); de Vos et al., *Science* 255 : 306-312 (1992)).

As to the selection of peptides or polypeptides bearing an antigenic epitope, it is well known that relatively short synthetic sequences that mimic part of a protein sequence are routinely capable of eliciting an antiserum that reacts with the partially mimicked protein. See for instance Sutcliffe, J. G., Shinnick, T. M., Green, N. and Learner, R. A., Antibodies that React with Predetermined Sites on Proteins, *Science* 219 : 660-666 (1983). Peptides capable of eliciting protein-reactive sera are frequently represented in the primary sequence of a protein, can be characterized by a set of simple chemical rules, and are confined neither to immunodominant regions of intact proteins (i.e. immunogenic epitopes) nor to the amino or carboxyl terminals.

Antigenic epitope-bearing peptides and polypeptides of the invention are therefore useful to raise antibodies, including monoclonal antibodies, that bind specifically to a polypeptide of the invention. Antigenic epitope-bearing peptides and polypeptides of the invention preferably contain a sequence of at least seven, more preferably at least nine and most preferably between about 15 to about 30 amino acids contained within the amino acid sequence of a polypeptide according to the invention.

The epitope-bearing peptides and polypeptides of the invention can be prepared by any conventional means, for instance by the method described in document US Patent n° 4,631,211.

KING-1 polypeptides of the invention and the epitope-bearing fragments thereof can be combined with parts of the constant domain of immunoglobulins (IgG), resulting in chimeric polypeptides. These fusion proteins facilitate the purification and generally show an increased half life *in vivo*. See for instance Traunecker et al., *Nature* 331 : 84-86 (1988). Fusion proteins that have a disulfide-linked dimeric structure due to the IgG part can also be more efficient in binding and neutralizing other molecules than the monomeric KING-1 protein or protein fragment thereof (Fountoulakis et al., *J. Biochem*. 270 : 3958-3964 (1995)).

The KING-1 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the KING-1 polypeptides or variants, mutated forms, derivatives thereof as hereabove mentioned are also included in the invention. A fragment is a polypeptide having an amino acid sequence that entirely is the same as part, but not all, of the amino acid sequence of the aforementioned KING-1 polypeptides. As with KING-1 polypeptides, fragments may be "free-standing," or comprised within a larger polypeptide of which they form a part or region, most preferably as a single continuous region.

Preferred fragments include, for example, truncation polypeptides having the amino acid sequence of KING-1 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate KING-1 activity, including those with a similar activity or an improved activity, or with a decreased undesirable activity. Also included are those that are antigenic or immunogenic in an animal, especially in a human.

Preferably, all of these polypeptide fragments retain the biological activity of KING-1, including antigenic activity.

The KING-1 polypeptides of the invention can be prepared in any suitable manner. Such polypeptides include isolated naturally occurring polypeptides, recombinantly produced polypeptides, or polypeptides produced by a combination of these methods.

In one embodiment, the KING-1 polypeptides of the invention can be prepared by chemical synthesis, for example by using non natural and/or modified amino acids. Thus, it can be advantageous to use non natural amino acids, for example D-amino acids, to enhance life time of the polypeptides, or analogs of amino acids, for example sulfurized forms of amino acids.

Methods for preparing such polypeptides are known in the art. For example, chemical synthesis of the polypeptides of the invention can be carried out by using a solid phase process. In such method, the C-terminal amino acid is fixed onto an inert solid support and comprises protective groups (e.g. BOC or FMOC) of the alpha-amino moiety. At the end of this step, the protective group is removed and a second amino acid, optionally protected in a similar manner, is fixed. The N-terminal protective groups are removed after each amino acid is fixed, protective groups possibly present on the lateral chains being conserved. When the peptide chain is synthesized, the peptide is cleaved from the support and the remaining protective groups are removed. Coupling can be carried out by using N,N'-diisopropyl-carbodiimine (DIC), among others, in a solvent of DMF or DCM. This solid phase synthesis is described in for example Stewart J. M. et al., Solid Phase Peptides Synthesis. Pierce Chem. Company, Rockford, 111, 2^{nd} Ed., (1984).

Other polypeptides according to the invention comprise the amino acid sequence of the KING-1 polypeptide which possesses the complete amino acid sequence encoded by the cDNA clone contained in ATCC deposit number PTA-1770, PTA-1771 or PTA-1772 as discussed below, as well as polypeptides which comprise the amino acid sequence of the mature KING-1 polypeptide which possesses the complete amino acid sequence encoded by the cDNA clone contained in ATCC deposit number PTA-1770, PTA-1771 or PTA-1772.

In another aspect, the invention provides a peptide or polypeptide comprising an epitope-bearing portion of a polypeptide of the invention. The epitope of this polypeptide portion is an immunogenic or antigenic epitope of a polypeptide described in the invention. An immunogenic epitope is defined as a part of a protein that elicits an antibody response when the whole protein is the immunogen. On the other hand, a region of a protein molecule to which an antibody can bind is defined as an antigenic epitope.

Another aspect of the invention relates to KING-1 polynucleotides.

Unless otherwise indicated, all nucleotide sequences determined by sequencing a DNA molecule herein have been determined by using an automated DNA sequencer, and all amino acid sequences of polypeptides encoded by DNA sequences determined herein have been predicted by translation of a DNA sequence determined as above. Therefore, as is known in the art for any DNA sequence determined by this approach, any nucleotide sequence may contain some errors. Nucleotide sequences determined by automation are typically at least about 90 % identical, more typically at least about 95 % to about 99.9 % identical to the actual nucleotide sequence of the sequenced DNA molecule.

KING-1 polynucleotides include isolated polynucleotides which encode the KING-1 polypeptides and fragments, and polynucleotides closely related thereto, e.g. variants, derivatives, mutated forms. More specifically, KING-1 polynucleotides of the invention include a polynucleotide comprising the nucleotide sequence set forth in SEQ ID NO: 1, SEQ ID NO: 2 or SEQ ID NO: 3 encoding a KING-1 polypeptide of SEQ ID NO: 4, SEQ ID NO: 5 or SEQ ID NO: 6 respectively, and a polynucleotide having the particular sequence of SEQ ID NO: 1, SEQ ID NO: 2 or SEQ ID NO: 3. KING-1 polynucleotides further include a polynucleotide comprising a nucleotide sequence that has at least 80% identity to a nucleotide sequence encoding the KING-1 polypeptides of SEQ ID NO: 4, SEQ ID NO: 5 or SEQ ID NO: 6 over its entire length, and a polynucleotide that is at least 80% identical to that having SEQ ID NO: 4, SEQ ID NO: 5 or SEQ ID NO: 6 over its entire length. In this regard, polynucleotides at least 90% identical are particularly preferred, and those with at least 95% identity are especially preferred. Furthermore, those with at least 97% identity are highly preferred and those with at least 98-99% identity are most highly preferred, those with at least 99% identity being the most preferred. Also included under KING-1 polynucleotides are nucleotide sequences which have sufficient identity to the nucleotide sequence contained in SEQ ID NO: 1, SEQ ID NO: 2 or SEQ ID NO: 3 or contained in the cDNA insert of the plasmid deposited with the ATCC Deposit number PTA-1770, PTA-1771 or PTA-1772 discussed below to hybridize under conditions useable for amplification or for use as a probe or marker. Moreover, KING-1 polynucleotides include a nucleotide sequence having at least 80% identity to a nucleotide sequence encoding the KING-1 polypeptide, either in a mature form or not, expressed by the cDNA insert deposited at the ATCC with Deposit Number PTA-1770, PTA-1771 or PTA-1772, and a nucleotide sequence comprising at least 15 contiguous nucleotides of such cDNA insert. In this regard, polynucleotides at least 90% identical are particularly preferred, and those with at least 95% identity are especially preferred. Furthermore, those with at least 97% identity are highly preferred and those with at least 98-99% identity are most highly preferred, those with at least 99% identity being the most preferred. The invention also provides polynucleotides which are complementary to all the KING-1 polynucleotides according to the invention.

A deposit containing a human KING-1 cDNA has been deposited with the American Type Culture Collection (ATCC), 10801 University Bld, Manassas, VA 20110-2209, USA, on April 27, 2000, and assigned ATCC Deposit Number PTA-1770. The deposited material (clone) is plasmid pcDNA4A which can be obtained from Invitrogen, Inc. that further contains the full length human cDNA of KING-1, referred to as "hKING-1" upon deposit. The cDNA insert is within EcoRI/Xhol sites in the vector. The nucleotide sequence of the polynucleotide contained in the deposited material, as well as the amino acid sequence of the polypeptide encoded thereby, are controlling in the event of any conflict with any description of sequences herein.

A deposit containing a human variant KING-1 cDNA has been deposited with the American Type Culture Collection (ATCC), 10801 University Bld, Manassas, VA 20110-2209, USA, on April 27, 2000, and assigned ATCC Deposit Number PTA-1772. The deposited material (clone) is plasmid pcDNA4A which can be obtained from Invitrogen, Inc. that further contains the full length human cDNA of KING-1, referred to as "hvKING-1" upon deposit. The cDNA insert is within EcoRI/Xhol sites in the vector. The nucleotide sequence of the polynucleotide contained in the deposited material, as well as the amino acid sequence of the polypeptide encoded thereby, are controlling in the event of any conflict with any description of sequences herein.

A deposit containing a rat KING-1 cDNA has been deposited with the American Type Culture Collection (ATCC), 10801 University Bld, Manassas, VA 20110-2209, USA, on April 27, 2000, and assigned ATCC Deposit Number PTA-1771. The deposited material (clone) is plasmid pcDNA4B which can be obtained from Invitrogen, Inc. that further contains the full length rat cDNA of KING-1, referred to as "rKING-1" upon deposit. The cDNA insert is within Notl sites in the vector. The nucleotide sequence of the polynucleotide contained in the deposited material, as well as the amino acid sequence of the polypeptide encoded thereby, are controlling in the event of any conflict with any description of sequences herein.

The deposit has been made under the terms of the Budapest Treaty on the international recognition of the deposit of micro-organisms for purposes of patent procedure.

The cDNA sequence of SEQ ID NO: 1 contains an open reading frame (nucleotide numbers 6 to 860) encoding a polypeptide of 284 amino acid residues of SEQ ID NO: 4 with a putative molecular weight of 32.0 Kd. The cDNA sequence of SEQ ID NO: 2 contains an open reading frame (nucleotide numbers 6 to 761) encoding a polypeptide of 251 amino acid residues of SEQ ID NO: 5 with a putative molecular weight of 28.3 Kd. The cDNA sequence of SEQ ID NO: 3 contains an open reading frame (nucleotide numbers 12 to 857) encoding a polypeptide of 281 amino acid residues of SEQ ID NO: 6 with a putative molecular weight of 31.1 Kd.

The polynucleotides of the invention encoding KING-1 may be obtained using standard cloning and screening, from a cDNA library derived from mRNA in cells of human kidney and testis using the expressed sequence tag (EST) analysis (Adams, M.D., *et al*. *Science* (1991) 252:1651-1656; Adams, M. D. *et al*., *Nature,* (1992) 355:632-634; Adams, M. D., *et al*., *Nature* (1995) 377 Supp:3-174). Polynucleotides of the invention can also be obtained from natural sources such as genomic DNA libraries or can be synthesized using well known and commercially available techniques.

The nucleotide sequence encoding KING-1 polypeptides of SEQ ID NO: 4, SEQ ID NO: 5 or SEQ ID NO: 6 may be identical to the polypeptide encoding sequence contained in SEQ ID NO: 1, SEQ ID NO: 2 or SEQ ID NO: 3 respectively, or it may be a different sequence which, as a result of the redundancy (degeneracy) of the genetic code, also encodes the polypeptides of SEQ ID NO: 4, SEQ ID NO: 5 or SEQ ID NO: 6.

As indicated, the invention also provides the mature form of the KING-1 polypeptide of the invention. According to the signal hypothesis, proteins secreted by mammalian cells have a signal or secretory leader sequence which is cleaved from the mature protein once export of the growing protein chain across the rough endoplasmic reticulum has been initiated. Most mammalian cells and even insect cells cleave secreted proteins with the same specificity. However, in some cases, cleavage of a secreted protein is not entirely uniform, which results in two or more mature species of the protein. Further, it has long been known that the cleavage specificity of a secreted protein is ultimately determined by the primary structure of the complete protein, i.e. its amino acid sequence. Therefore, the invention provides a nucleotide sequence encoding the mature KING-1 polypeptide having the amino acid sequence encoded by the cDNA clone contained in the host identified as ATCC Deposit PTA-1770, PTA-1771 or PTA-1772. By the mature KING-1 polypeptide having the amino acid sequence encoded by the cDNA clone contained in the host identified as ATCC Deposit PTA-1770, PTA-1771 or PTA-1772 is meant the mature form of the KING-1 polypeptide produced by expression in a mammalian cell of the complete open reading frame encoded by the human DNA sequence of the clone contained in the vector in the deposited host. As indicated, the mature KING-1 polypeptide having the amino acid sequence encoded by the cDNA clone contained in ATCC Deposit PTA-1770, PTA-1771 or PTA-1772 may or may not differ from the predicted "mature" KING-1 protein represented in SEQ ID NO: 4, SEQ ID NO: 5 or SEQ ID NO: 6, depending on the accuracy of the predicted cleavage site based on computer analysis.

Methods for predicting whether a protein has a secretory leader as well as the cleavage site of this leader sequence are available. For instance, the methods of McGeoch (*Virus Res*. 3:271-286 (1985)) and von Heinje (*Nucleic Acids Res*. 14:4683-4690 (1986)) can be used.

When the polynucleotides of the invention are used for the recombinant production of KING-1 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al*., *Proc. Natl*. *Acad*. *Sci. USA* (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding KING-1 variants comprising the amino acid sequence of KING-1 polypeptides of SEQ ID NO: 4, SEQ ID NO: 5 or SEQ ID NO: 6 in which several, for example 5-10, 1-5, 1-3, 1-2 or 1 amino acid residues are substituted, deleted or added, in any combination.

The invention is further directed to fragments of the isolated nucleic acid molecules described herein. By a fragment of an isolated nucleic acid molecule having the nucleotide sequence of the deposited cDNA or the nucleotide molecule having the nucleotide sequence shown in SEQ ID NO: 1, SEQ ID NO: 2 or SEQ ID NO: 3 is intended fragments of at least about 15 nucleotides, and preferably of at least about 20 nucleotides, more preferably of at least about 30 nucleotides, even more preferably of at least about 40 nucleotides, which are useful as diagnostic probes and primers. By a fragment of at least 20 nucleotides in length, for example, are intended fragments which include 20 or more contiguous bases from the nucleotide sequence of the deposited cDNA or from the sequence shown in SEQ ID NO: 1, SEQ ID NO: 2 or SEQ ID NO: 3, deleted from a continuous series of residues including the 5' end or 3' end or both.

The invention further relates to polynucleotides that hybridize to the herein above-described sequences. In this regard, the invention especially relates to polynucleotides which hybridize under stringent conditions to the herein above-described polynucleotides. As herein used, the term "stringent conditions" means hybridization will occur only if there is at least 95% and preferably at least 97% identity between the sequences.

Polynucleotides of the invention, which are identical or sufficiently identical to a nucleotide sequence contained in SEQ ID NO: 1, SEQ ID NO: 2 or SEQ ID NO: 3 or a fragment thereof, or to the cDNA insert in the plasmid deposited at the ATCC with Deposit Number PTA-1770, PTA-1771 or PTA-1772 or a fragment thereof, may be used as hybridization probes for cDNA and genomic DNA, to isolate full-length cDNAs and genomic clones encoding KING-1 and to isolate cDNA and genomic clones of other genes that have a high sequence similarity to the KING-1 gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, obtaining a polynucleotide encoding KING-1 polypeptide comprises the steps of screening an appropriate library under stringent hybridization conditions with a labeled probe having the SEQ ID NO: 1, SEQ ID NO: 2 or SEQ ID NO: 3 or a fragment thereof; and isolating full-length cDNA and genomic clones containing said polynucleotide sequence. Such hybridization techniques are well known to those of skill in the art. Stringent hybridization conditions are as defined above or alternatively conditions under overnight incubation at 42°C in a solution comprising: 50% formamide, 5xSSC (150 mM NaCI, 15 mM trisodium citrate), 50 mM sodium phosphate (pH 7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the invention may be employed as research reagents and materials for discovery of treatments and diagnostics for animal and human diseases.

The invention also relates to recombinant vectors which comprise a polynucleotide or polynucleotides according to the invention and expression system capable of producing the KING-1 polypeptide when said expression system is present in a compatible host cell, host cells which are genetically engineered with vectors of the invention. The invention also relates to the production of polypeptides of the invention by recombinant techniques. Cell-free translation systems can also be employed to produce such proteins using RNAs derived from the DNA constructs of the invention.

For recombinant production, host cells can be genetically engineered to incorporate expression systems or portions thereof for polynucleotides of the invention. Introduction of polynucleotides into host cells can be effected by methods described in many standard laboratory manuals, such as Davis et al., *Basic Methods in Molecular Biology* (1986) and Sambrook et al., *Molecular Cloning : a Laboratory Manual,* 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

The expression vectors will preferably include at least one selectable marker. Such markers include dihydrofolate reductase or neomycin resistance for eukaryotic cell culture and tetracycline or ampicillin resistance genes for culturing *E. coli* and other bacteria.

The DNA insert should preferably be linked to an appropriate promoter, such as the bacterial phage lambda PL and RP, T3 and T7, *E*. *coli lac*, *trp* and *tac* promoters, the eukaryotic SV40 early and late promoters, CMV immediate early promoters, eukaryotic promoters from retroviral LTRs, such as those of the Rous Sarcoma Virus (RSV) to name a few. Other suitable promoters are available for the skilled artisan.

The expression constructs will further contain sites for transcription initiation, termination and, in the transcribed region, a ribosome binding site for translation. The coding region of the mature transcripts expressed by the constructs will preferably include a translation initiating at the beginning and a termination codon (UAA, AGA or UAG) appropriately positioned at the end of the polypeptide to be translated.

Representative examples of appropriate hosts comprise bacterial cells, such as streptococci, staphylococci, *E. coli*, *Streptomyces, Salmonella typhimurium* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. Generally, any system or vector suitable to maintain, propagate or express polynucleotides, for producing a polypeptide in a host, may be used.

Among vectors preferred for use in bacteria are pGE70, pGE60 and pG-9, available form Qiagen; Phagescript vectors, pBS vectors, Bluescript vectors, pNH8A, pNH16a, pNH18A, pNH46A, available from Stratagene, ptrc99a, pKK223-3, pDR540, pRIT5 available from Pharmacia.

Among preferred eukaryotic vectors are pWLNEO, pSV2CAT, pOG44, pXT1 and pSG available from Stratagene; pSVK3, pBPV, pMSG and pSVL available from Pharmacia, pCDNA3, pCDNA3.1 and pTracer™ available from Invitrogene. Other suitable vectors will be readily apparent to the skilled in the art.

The appropriate nucleotide sequence may be inserted into an expression system by any of a variety of well-known and routine techniques, such as, for example, those set forth in Sambrook *et al*., *Molecular Cloning, a Laboratory Manual* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the KING-1 polypeptide is to be expressed for use in screening assays, generally, it is preferred that the polypeptide be produced at the surface of the cell. In this event, the cells may be harvested prior to use in the screening assay. If KING-1 polypeptide is secreted into the medium, the medium can be recovered in order to recover and purify the polypeptide; if produced intracellularly, the cells must first be lysed before the polypeptide is recovered.

KING-1 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

The polypeptide may be expressed in a modified form, such as a fusion protein, and may include not only secretion signals, but also additional heterologous functional regions. For instance, a region of additional amino acids, particularly charged amino acids, may be added to the N-terminus of the polypeptide to improve stability and persistence in the host cell, during purification or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to solubilize proteins. For example, EP-A-0 464 533 discloses fusion proteins comprising various portions of constant region of immunoglobulin molecules together with another human protein or part thereof. In many cases, the Fc part in a fusion protein is advantageous for use in therapy and diagnosis. On the other hand, for some uses, it would be desirable to delete the Fc part after the fusion protein has been expressed, detected and purified. For instance, it is the case when Fc portion proves to be a hindrance to use in therapy and diagnosis, for example when the fusion protein is to be used as antigen for immunizations.

It is deemed that mammals with certain diseases or susceptibility to certain diseases express significantly altered, enhanced or diminished levels of the KING-1 protein and mRNA encoding the KING-1 protein, when compared to a corresponding "standard" mammal, i.e. a mammal of the same species not having or not presenting a susceptibility to the disease. Further, it is deemed that significantly altered, enhanced or diminished levels of the KING-1 protein can be detected in certain body fluids (e.g. bone marrow, lymph fluid, blood, sera, plasma, saliva, urine, synovial fluid and spinal fluid) from mammals with the disease or presenting a susceptibility to the disease when compared to sera from mammals of the same species not having or not presenting a susceptibility to the disease. Thus, the invention provides a diagnostic method which involves assaying the expression level of the gene encoding a KING-1 polypeptide of the invention in mammalian cells or body fluid and comparing the gene expression level with a standard KING-1 polypeptide expression level, whereby an altered, enhanced or diminished expression level of the gene with respect to the standard is indicative of the disease or of a susceptibility to the disease. Preferred mammals include monkeys, apes, cats, dogs, cows, pigs, horses, rabbits and humans.

Diseases which can be diagnosed include but are not limited to ischemic renal failure, glomerulonephritis, chronic renal failure, diabetic nephropathy, cardiac ischemia, myocardial infarction, hypertension, congestive heart failure, inflammatory and proliferative diseases, among others, e.g. through detection of mutation in the Kidney Injury Novel Gene-1 of the invention.

By "assaying the expression level of the gene encoding a KING-1 protein" is intended qualitatively or quantitatively measuring or estimating the level of a KING-1 protein or the level of the mRNA encoding a KING-1 protein in a first biological sample either directly (e.g. by determining or estimating absolute protein level or mRNA level) or relatively (e.g. by comparing with the KING-1 protein level or mRNA level in a second biological sample).

Preferably, the KING-1 protein level or mRNA level in the first biological sample is measured or estimated and compared with a standard KING-1 protein level or mRNA level, the standard being taken from a second biological sample obtained from an individual not having (nor presenting a susceptibility to) the disease. As will be appreciated , once a standard KING-1 protein level or mRNA level is known, it can be used repeatedly as a standard for comparison.

By "biological sample" is intended any biological sample, either tissue sample or body fluid, obtained from an individual, cell line, tissue culture, or other source which contains KING-1 protein or mRNA.

Total cellular RNA can be isolated from a biological sample using the single-step guanidinium-thiocyanate-phenol-chloroform method described in Chomczynski and Sacchi, *Anal*. *Biochem*. 162 : 156-159 (1987). Levels of mRNA encoding the KING-1 polypeptide are then assayed using any appropriate method. These include Northern Blot analysis (Harada et al., *Cell* 63 : 303-312 (1990)), S1 nuclease mapping (Fujita et al., *Cell* 49 : 357-367 (1987)), the polymerase chain reaction (PCR), reverse transcription in combination with the polymerase chain reaction (RT-PCR) (Fujita et al., *Cell* 49 : 357-367 (1987)), and reverse transcription in combination with the ligase chain reaction (RT-LCR).

Assaying KING-1 protein levels in a biological sample can occur using antibody-based techniques. For example, KING-1 protein expression in tissues can be studied with classical immunohistological methods (Jalkanen, M., et al., *J. Cell. Biol*. 101 : 976-985 (1985); Jalkanen, M., et al., *J. Cell. Biol*. *105 : 3087-3096 (1987))*. Other antibody-based methods useful for detecting KING-1 gene expression include immunoassays, such as the enzyme linked immunosorbent assay (ELISA) and the radioimmunoassay (RIA).

Suitable labels are known in the art and include enzyme labels, such as glucose oxidase, and radioisotopes, such as iodine (¹²⁵I, ¹²¹I), carbon (¹⁴C), sulfur (³⁵S), tritium (³H), indium (¹¹²In), and technetium (^{99m}Tc), and fluorescent labels, such as fluorescein and rhodamine and biotin.

In addition, the invention provides a diagnostic assays for diagnosing or determining a susceptibility to ischemic renal failure, glomerulonephritis, chronic renal failure, diabetic nephropathy, cardiac ischemia, myocardial infarction, hypertension, congestive heart failure, inflammatory and proliferative diseases, among others through detection of mutation in the KING-1 gene by the methods described below.

The diagnostic assays of the invention comprises:
(a) a Kidney Injury Novel Gene-1 polynucleotide, preferably the nucleotide sequence of SEQ ID NO: 1, SEQ ID NO: 2 or SEQ ID NO: 3, or a fragment thereof;
(b) a nucleotide sequence complementary to that of SEQ ID NO: 1, SEQ ID NO: 2 or SEQ ID NO: 3;
(c) a Kidney Injury Novel Gene-1 polypeptide, preferably the polypeptide of SEQ ID NO: 4, SEQ ID NO: 6 or SEQ ID NO: 6, or a fragment thereof; or
(d) an antibody against a Kidney Injury Novel Gene-1 polypeptide, preferably to the polypeptide of SEQ ID NO: 4, SEQ ID NO:5 or SEQ ID NO: 6.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled KING-1 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al*., *Science* (1985) 230:1242. Sequence changes at specific locations may also be revealed by nuclease protection assays, such as RNase and S1 protection or the chemical cleavage method. See Cotton *et al*., *Proc*. *Natl. Acad*. *Sci. USA* (1985) 85: 4397-4401. In another embodiment, an array of oligonucleotides probes comprising KING-1 nucleotide sequence or fragments thereof can be constructed to conduct efficient screening of e.g., genetic mutations. Array technology methods are well known and have general applicability. They can be used to address a variety of questions in molecular genetics including gene expression, genetic linkage, and genetic variability. (See for example: M.Chee et al., *Science,* Vol 274, pp 610-613 (1996)).

The nucleotide sequences of the invention are also valuable for chromosome identification. The sequence is specifically targeted to and can hybridize with a particular location on an individual human chromosome. The mapping of relevant sequences to chromosomes according to the invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease.

The polypeptides of the invention or their fragments or analogs thereof, or cells expressing them can also be used as immunogens to produce antibodies or fragments of antibodies immunospecific for the KING-1 polypeptides. The term "immunospecific" means that the antibodies have substantial greater affinity for the polypeptides of the invention than their affinity for other related polypeptides.

Antibodies generated against the KING-1 polypeptides can be obtained by administering the polypeptides or epitope-bearing fragments, analogs or cells to an animal, preferably a non human, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al*., *Immunology Today* (1983) 4:72) and the EBV-hybridoma technique (Cole *et al*., Monoclonal Antibodies and Cancer Therapy, pp. 77-96, Alan R. Liss, Inc., 1985).

Techniques for the production of single chain antibodies (U.S. Patent No. 4,946,778) can also be adapted to produce single chain antibodies to polypeptides of the invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against KING-1 polypeptides may also be employed to treat ischemic renal failure, glomerulonephritis, chronic renal failure, diabetic nephropathy, cardiac ischemia, myocardial infarction, hypertension, congestive heart failure, inflammatory and proliferative diseases, among others.

Another aspect of the invention relates to a method for inducing an immunological response in a mammal which comprises inoculating the mammal with KING-1 polypeptide, or a fragment thereof, adequate to produce antibody and/or T cell immune response to protect said animal from ischemic renal failure, glomerulonephritis, chronic renal failure, diabetic nephropathy, cardiac ischemia, myocardial infarction, hypertension, congestive heart failure, inflammatory and proliferative diseases, among others. Yet another aspect of the invention relates to a method of inducing an immunological response in a mammal which comprises, delivering KING-1 polypeptide via a vector directing expression of KING-1 polynucleotide *in vivo* in order to induce such an immunological response and to produce antibody to protect said animal from diseases.

A further aspect of the invention relates to an immunological/vaccine formulation (composition) which, when introduced into a mammalian host, induces an immunological response in that mammal to a KING-1 polypeptide wherein the composition comprises a KING-1 polypeptide or KING-1 gene. The vaccine formulation may further comprise a suitable carrier. Since KING-1 polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation isotonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in-water systems and other systems known in the art. The dosage will depend on the specific activity of the vaccine and can be readily determined by routine experimentation.

The KING-1 polypeptide of the invention may be employed in a screening process for candidate compounds which, directly or indirectly, activate (agonists) or inhibit activation (antagonists, otherwise called inhibitors) of the KING-1 polypeptide of the invention. Thus, polypeptides of the invention may also be used to assess the binding of small molecule substrates and ligands in, for example, cells, cell-free preparations, chemical libraries, and natural product mixtures. These substrates and ligands may be natural substrates and ligands or may be structural or functional mimetics. See Coligan *et al*., *Current Protocols in Immunology* 1(2): Chapter 5 (1991).

KING-1 polypeptides are deemed to be responsible for many biological functions, including many pathologies. Accordingly, it is desirous to find compounds and drugs which stimulate KING-1 on the one hand or which can inhibit the function of KING-1 on the other hand. In general, agonists are employed for therapeutic and prophylactic purposes for such conditions as ischemic renal failure, glomerulonephritis, chronic renal failure, diabetic nephropathy, cardiac ischemia, myocardial infarction, hypertension, congestive heart failure, inflammatory and proliferative diseases. Antagonists may be employed for a variety of therapeutic and prophylactic purposes for such conditions as ischemic renal failure, glomerulonephritis, chronic renal failure, diabetic nephropathy, cardiac ischemia, myocardial infarction, hypertension, congestive heart failure, inflammatory and proliferative diseases.

In general, such screening procedures involve producing appropriate cells which express the receptor polypeptide of the invention on the surface thereof. Such cells include cells from mammals, yeast, *Drosophila* or *E. coli*. Cells expressing the receptor (or cell membrane containing the expressed receptor) are then contacted with a test compound to observe binding, stimulation or inhibition of a functional response.

The assays may simply test the binding of a candidate compound, wherein adherence to the cells bearing the receptor is detected by means of a label directly or indirectly associated with the candidate compound or in an assay involving competition with a labeled competitor. Further, these assays may test whether the candidate compound results in a signal generated by activation of the receptor, using detection systems appropriate to the cells bearing the receptor at their surfaces. Inhibitors of activation are generally assayed in the presence of a known agonist and the effect on activation by the agonist in the presence of the candidate compound is observed. Standard methods for conducting such screening assays are well understood in the art.

Examples of potential KING-1 antagonists include antibodies or, in some cases, oligonucleotides or proteins which are closely related to the ligand of the KING-1, e.g., a fragment of the ligand, or small molecules which bind to the receptor but do not elicit a response, so that the activity of the receptor is prevented.

Thus in another aspect, the invention relates to a screening kit for identifying agonists, antagonists, ligands, receptors, substrates, enzymes, etc. for Kidney Injury Novel Gene-1 polypeptides; or compounds which decrease or enhance the production of Kidney Injury Novel Gene-1 polypeptides, which comprises:
(a) a Kidney Injury Novel Gene-1 polypeptide, preferably that of SEQ ID NO: 4, SEQ ID NO: 5 or SEQ ID NO: 6;
(b) a recombinant cell expressing a Kidney Injury Novel Gene-1 polypeptide, preferably that of SEQ ID NO: 4, SEQ ID NO: 5 or SEQ ID NO: 6;
(c) a cell membrane expressing a Kidney Injury Novel Gene-1 polypeptide; preferably that of SEQ ID NO: 4, SEQ ID NO: 5 or SEQ ID NO: 6; or
(d) an antibody against a Kidney Injury Novel Gene-1 polypeptide, preferably that of SEQ ID NO: 4, SEQ ID NO: 5 or SEQ ID NO: 6.

The invention provides methods of treating abnormal conditions related to both an over-activity or an insufficient activity of KING-1.

In a first aspect, the invention provides a method of treating an individual in need of an altered, diminished or inhibited level of KING-1 activity or expression, comprising administering to such an individual a pharmaceutical composition comprising an effective amount of one or several active ingredients capable of altering or diminishing the KING-1 activity.

In that case, several approaches are available. One approach comprises administering as active ingredient an inhibitor compound (antagonist) identifiable by the method as above described, with a pharmaceutically acceptable carrier, in an amount effective to inhibit the function of King Injury Novel Gene-1, e.g. by blocking the binding of ligands, substrates, enzymes, receptors, etc, or by inhibiting a second signal, and thereby alleviating the abnormal condition.

In another approach, soluble forms of KING-1 polypeptides still capable of binding the ligand, substrate, enzyme, receptor, etc in competition with endogenous KING-1 may be administered as active ingredient. Typical embodiments of such competitors comprise fragments of the KING-1 polypeptide.

In still another approach, expression of the gene encoding endogenous KING-1 can be inhibited using expression blocking techniques. Such known techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al*., *Nucleic Acids Res.* (1979) 6:3073; Cooney *et al*., *Science* (1988) 241:456; Dervan *et al*., *Science* (1991) 251:1360. These oligomers can be administered *per* se or the relevant oligomers can be expressed *in vivo*.

In a second aspect, the invention provides a method of treating an individual in need of an increased level of KING-1 activity or expression, comprising administering to such an individual a pharmaceutical composition comprising an effective amount of one or several active ingredients capable of increasing the KING-1 activity.

In that case, several approaches are also available. One approach comprises administering as active ingredient a therapeutically effective amount of a compound which activates KING-1, i.e., an agonist identifiable by the method as described above, in combination with a pharmaceutically acceptable carrier, to thereby alleviate the abnormal condition.

Alternatively, gene therapy may be employed to effect the endogenous production of KING-1 by the relevant cells in the subject. For example, a polynucleotide of the invention may be engineered for expression in a replication defective retroviral vector, as discussed above. The polynucleotide constitutes in that case the active ingredient. The retroviral expression construct may then be isolated and introduced into a packaging cell transduced with a retroviral plasmid vector containing RNA encoding a polypeptide of the invention such that the packaging cell now produces infectious viral particles containing the gene of interest. These producer cells may be administered to a subject for engineering cells *in vivo* and expression of the polypeptide *in vivo*. For overview of gene therapy, see Chapter 20, Gene Therapy and other Molecular Genetic-based Therapeutic Approaches, (and references cited therein) in *Human Molecular Genetics,* T. Strachan and A. P. Read, BIOS Scientific Publishers Ltd. (1996).

In another aspect, the invention provides pharmaceutical compositions for preventing and/or treating in a subject a disease or susceptibility to a disease related to activity of KING-1.

Pharmaceutical compositions of the invention are intended, but not limited to, the treatment and/or prevention of ischemic renal failure, glomerulonephritis, chronic renal failure, diabetic nephropathy, cardiac ischemia, myocardial infarction, hypertension, congestive heart failure, inflammatory and proliferative diseases, among others.

In a first aspect, the pharmaceutical compositions of the invention for preventing and/or treating in a subject a disease or susceptibility to a disease related to over-activity or over-expression of KING-1 may comprise a therapeutically effective amount of at least an antagonist of KING-1 or a derivative or homologue thereof as defined above, and/or at least a nucleic acid molecule that inhibits the expression of the nucleotide sequence encoding said receptor (antisense sequence) and/or at least a peptide which competes with said receptor, as discussed above.

In a second aspect, the pharmaceutical compositions of the invention for preventing and/or treating in a subject a disease or susceptibility to a disease related to under-activity or under-expression of KING-1 may comprise a therapeutically effective amount of at least an agonist of KING-1 or a derivative or homologue thereof as defined above, and/or at least a polynucleotide according to the invention and/or at least a nucleotide sequence according to the invention able to express *in vivo* a KING-1 polypeptide of the invention.

The pharmaceutical compositions in which at least a polypeptide according to the invention is used may comprise the recombinant vectors of the invention, able to express *in vivo* a polypeptide according to the invention. Polypeptides according to the invention may be also used "naked", i.e. without being inserted into a vector. It has been shown that some DNA or RNA sequences can be expressed in some tissues without the aid of an expression vector.

Pharmaceutical compositions according to the invention, for example those which comprise polypeptides of the invention, such as the soluble form of KING-1 polypeptides, agonists and antagonists or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, intrasternal, intraarterial or intraperitoneal, can be used. Alternative means for systemic administration include transmucosal and transdermal administration using penetrants such as bile salts or fusidic acids or other detergents. In addition, if properly formulated in enteric or encapsulated formulations, oral, rectal, intravaginal administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 1 µg/kg/day to 10 mg/kg/day of subject. More preferably, this dose is at least between 0.01 mg/kg/day and 1 mg/kg/day. Variations in the needed dosage are to be expected in view of the nature of the compounds used and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide of the invention, such as a DNA or RNA, to encode a polypeptide *ex vivo*, and for example, by the use of a retroviral plasmid vector. The cells are then introduced into the subject.

The examples below are carried out using standard techniques, which are well known and routine to those of skill in the art, except where otherwise described in detail.

### Example 1.

### Animal models and identification of a new gene up-regulated after renal ischemia and reperfusion.

Two animal model systems have been studied to provide targets for intervention in acute renal failure. An ischemia-reperfusion model of acute renal failure was developed in Fischer 344 and Lewis rats, by occluding both renal arteries for 30 minutes followed by various periods of reperfusion. Severity of acute renal failure was monitored at the different times of renal reperfusion, by measuring plasma creatinine and blood urea nitrogen (BUN) levels. In order to identify genes induced by renal injury, a subtractive cDNA library has been constructed starting from kidneys recovered after 24 h of reperfusion from ischemic and sham operated Fischer 344 rats, using the Suppression Subtractive Hybridization method (Diatchenko L. et al., 1996 *Proc*. *Natl*. *Acad*. *Sci. USA* 93:6025-6030) of the PCR-Select cDNA Subtraction Kit (Clontech Inc.).

Subtracted cDNAs were then cloned into pTAdv. vector (Clontech Inc.). Individual colonies were randomly picked and organized into 96-well microtiter plates. Glycerol stocks were kept at -80°C. For macroarrays preparation, the cDNA clone inserts from the subtracted library were amplified by PCR using AmpliTaq polymerase (Perkin-Elmer) and vector-specific primers. PCR products were spotted onto Nylon filters (Hybond N⁺, Amersham, UK) using a Biomek 2000 robot (Beckman, France), at a density of 384 spots (4 x 96 microtiter plates) per 8x12 cm filter. DNA was denatured by incubation of the filters in NaOH 0.5 M, NaCl 1.5 M, followed by a neutralization step in 5xSSC, and fixed to the membrane by baking at 80°C for 2 h. Control cDNAs specific for various house-keeping genes (β-actin, GAPDH, ubiquitin), as well as rat genomic DNA were spotted at specific positions on the filters. Membranes were prepared in batches and stored at 4°C until use.

Differential screening was performed using the following methods: Single strand radiolabeled complex cDNA probes were generated from ischemic and sham kidneys (24 h of reperfusion) by reverse transcription of 1 µg of polyA⁺ RNA using Superscript II reverse transcriptase (Life Technologies) and random hexamers (Perkin Elmer). Labeling was performed by incorporating 50 µCi of [α-³²P]-dATP (Dupont NEN; 3000 Ci/mmole). Unincorporated nucleotides were separated from the labeled probe by filtration on a Nuc Trap Pusch column (Stratagene). Filters were prehybridized for 6 h at 68°C in Clontech ExpressHyb Solution added with 100 µg/ml of denatured salmon sperm DNA. Complex probes were denatured for 20 min. at 68°C in NaOH 0.1 M, EDTA 1mM, neutralised for 10 min. at 68°C in NaH₂PO₄ 0.5 M, pH7.0, Cot1 DNA (0.01 µg/µl). Hybridizations were performed overnight at 68°C in ExpressHyb solution with 4.10⁶ cpm/ml of ³² P-radiolabeled probe. After hybridization, a final washing is carried out (0.1xSSC / 0.5% SDS) at 68°C. The macroarrays were then exposed to phosphor screens (Molecular Dynamics, CA) for 5 days and scanned onto a Phosphorlmager STORM 820 (Molecular Dynamics) at a resolution of 100 µm. Quantification of hybridization signal intensities was carried out using the Image Quant software (version 3.1) (Molecular Dynamics).

Among a number of genes found to be activated in ischemic kidneys, a particular gene, called KING-1, was significantly over-expressed after renal ischemia whereas its transcript expression on a multiple rat normal tissue Northern blot appeared in heart, testis, liver, skeletal muscle, lung, kidney.

### Example 2.

### Cloning of rat and human KING-1 cDNAs.

A combination of ClonCapture and 5' RACE technologies (Clontech, Inc.) was used in order to isolate a full-length cDNA containing the entire coding sequence of rat KING-1 gene.

A human KING-1 cDNA was cloned by hybridization of Human Universal cDNA Library membranes (HUCL) from Stratagene. It corresponded to a splice variant lacking 99 bp inside the coding region when aligned to the rat cDNA sequence. A full-length human KING-1 cDNA was reconstituted using the HUCL clone and Reverse-transcription PCR approach.

### Example 3.

### Northern blot analysis of the temporal expression of KING-1 mRNA during the course of the development of ischemic acute renal failure.

KING-1 mRNA up-regulation following renal ischemia and reperfusion was confirmed by Northern blot hybridization. Fischer 344 and Lewis rats from ischemic and sham-operated groups were sacrified at different times following reperfusion, e.g 2 h, 4 h, 6 h, 24 h, 48 h, 72 h and 120 h. Both kidneys were rapidly excised, cleaned of surrounding tissues, snap frozen in liquid nitrogen and stored at -80°C until RNA extraction. Total RNAs were extracted from pools of frozen kidneys using RNeasy (QIAGEN). Poly-A⁺ RNAs were purified by chromatography on oligo-dT cellulose columns (Pharmacia). Equal amounts of polyA⁺ RNA (4 µg) were electrophoresed through formaldehyde - 1% agarose gels, transferred to Nylon membranes (BiodyneA, PALL), and fixed to the membrane by baking at 80°C for 2 h. cDNA probe for rat KING-1 was labeled with [³²P]-dCTP by random-priming (MegaPrime kit, Amersham, UK). Membranes were hybridized for 2 h at 68°C using ExpressHyb Hybridization Solution (Clontech). After hybridization, final washing conditions were 50°C with 0.1xSSC / 0.1% SDS.

Northern blot analysis showed that KING-1 mRNA was markedly increased in kidneys from Fischer 344 and Lewis rats starting from 6 h post-reperfusion.

## Claims

1. An isolated polynucleotide comprising a nucleotide sequence at least 80% identical to a nucleotide sequence selected from the group consisting of:
(a) the nucleotide sequence set forth in SEQ ID NO: 1, SEQ ID NO: 2 or SEQ ID NO: 3;
(b) a nucleotide sequence which comprises the KING-1 polypeptide encoding sequence contained in SEQ ID NO: 1, SEQ ID NO: 2 or SEQ ID NO: 3;
(c) a nucleotide sequence comprising the sequence located between nucleotide numbers 6 to 860 set forth in SEQ ID NO: 1, the nucleotide sequence comprising the sequence located between nucleotide numbers 6 to 761 set forth in SEQ ID NO: 2 or the nucleotide sequence comprising the nucleotide sequence located between nucleotide numbers 12 to 857 set forth in SEQ ID NO: 3;
(d) a nucleotide sequence encoding a polypeptide comprising amino acids from 1 to 284 set forth in SEQ ID NO: 4, the nucleotide sequence encoding a polypeptide comprising amino acids from 1 to 251 set forth in SEQ ID NO: 5 or the nucleotide sequence encoding a polypeptide comprising amino acids from 1 to 281 set forth in SEQ ID NO: 6;
(e) the nucleotide sequence encoding the KING-1 polypeptide expressed by the cDNA clone contained in ATCC deposit number PTA-1770, PTA-1771 or PTA-1772;
(f) the nucleotide sequence encoding the mature KING-1 polypeptide expressed by the cDNA clone contained in ATCC deposit number PTA-1770, PTA-1771 or PTA-1772;
(g) a nucleotide sequence able to hybridize with the nucleotide sequence set forth in SEQ ID NO: 1, SEQ ID NO: 2 or SEQ ID NO: 3 or with the cDNA clone contained in ATCC deposit number PTA-1770, PTA-1771 or PTA-1772;
(h) a nucleotide sequence complementary to any of the nucleotide sequences defined in (a), (b), (c), (d), (e), (f) or (g) above, as well as fragments, variants, derivatives, mutated forms of these nucleotide sequences.

2. Polynucleotide according to claim 1 which is DNA or RNA.

3. A DNA or RNA molecule constituting a recombinant vector, comprising an expression system which is capable of producing a KING-1 polypeptide when said expression system is present in a compatible host cell, wherein said DNA or RNA molecule comprises a polynucleotide according to claim 1 or 2.

4. A host cell comprising the expression system of claim 3.

5. A process for producing a KING-1 polypeptide, comprising the steps of transforming or transfecting a host cell with an expression system according to claim 3, culturing said host cell under appropriate culture conditions so that said host cell produces the KING-1 polypeptide, and recovering the polypeptide from the culture.

6. An isolated KING-1 polypeptide comprising an amino acid sequence which is at least 80% identical to a sequence selected from the group consisting of:
(a) the amino acid sequence set forth in SEQ ID NO: 4, SEQ ID NO: 5 or SEQ ID NO: 6;
(b) the amino acid sequence comprised between amino acids 1 to 284 of the sequence set forth in SEQ ID NO: 4, the amino acid sequence comprised between amino acids 1 to 251 of SEQ ID NO: 5 or the amino acid sequence comprised between 1 to 281 of the sequence set forth in SEQ ID NO: 6;
(c) the amino acid sequence of the KING-1 polypeptide having the complete amino acid sequence encoded by the cDNA clone contained in ATCC deposit number PTA-1770, PTA-1771 or PTA-1772;
(d) the amino acid sequence of the mature KING-1 polypeptide having the complete amino acid sequence encoded by the cDNA clone contained in ATCC deposit number PTA-1770, PTA-1771 or PTA-1772; as well as fragments, derivatives, variants or mutated forms of these amino acid sequences.

7. Polypeptide according to claim 6, wherein it is obtained by the process of claim 5 or by chemical synthesis.

8. A method for identifying agonists of KING-1 polypeptide, which comprises the steps of:
(a) contacting a host cell according to claim 4 with a candidate compound; and
(b) determining whether the candidate compound effects a signal generated by activation of the KING-1 polypeptide.

9. A method for identifying antagonists of KING-1 polypeptide, which comprises the steps of:
(a) contacting a host cell according to claim 4 with an agonist of KING-1 polypeptide; and
(b) determining whether the signal generated by said agonist is diminished in the presence of a candidate compound.

10. A process for diagnosing a disease or a susceptibility to a disease in a subject related to expression or activity of KING-1 polypeptide comprising the steps of:
(a) determining the presence or absence of a mutation in the nucleotide sequence encoding said KING-1 polypeptide in the genome of said subject; and/or
(b) analyzing for the presence or amount of said KING-1 polypeptide expression in a sample derived from said subject.

11. Pharmaceutical composition, for preventing and/or treating in a subject a disease or susceptibility to a disease related to an under-expression or under-activity of KING-1, said composition comprising a therapeutically effective amount of at least an agonist of KING-1 polypeptide or derivative or homologue thereof identified by the method of claim 8, and/or at least a polypeptide according to claim 6, and/or at least a nucleotide sequence able to express *in vivo* a KING-1 polypeptide according to claim 6.

12. Pharmaceutical composition for preventing and/or treating in a subject a disease or susceptibility to a disease related to over-activity or over-expression of KING-1, said composition comprising a therapeutically effective amount of at least an antagonist of KING-1 or a derivative or homologue thereof identified by the method of claim 9, and/or at least a nucleic acid molecule that inhibits the expression of the nucleotide sequence encoding KING-1 and/or at least a peptide which competes with KING-1.

13. Pharmaceutical composition according to claim 11 or 12, wherein it comprises a recombinant vector according to claim 3, able to express *in vivo* a polypeptide according to claim 6.

14. Pharmaceutical composition according to any of claims 11 to 13, wherein it further comprises at least a pharmaceutically acceptable carrier.

15. Pharmaceutical composition according to any of claims 11 to 14, for prevention and/or treatment of ischemic renal failure, glomerulonephritis, chronic renal failure, diabetic nephropathy, cardiac ischemia, myocardial infarction, hypertension, congestive heart failure, inflammatory and proliferative diseases.

16. A diagnostic assays for diagnosing or determining a susceptibility to ischemic renal failure, glomerulonephritis, chronic renal failure, diabetic nephropathy, cardiac ischemia, myocardial infarction, hypertension, congestive heart failure, inflammatory and proliferative diseases which comprises:
(a) a Kidney Injury Novel Gene-1 polynucleotide, preferably the nucleotide sequence of SEQ ID NO: 1, SEQ ID NO: 2 or SEQ ID NO: 3 or a fragment thereof;
(b) a nucleotide sequence complementary to that of SEQ ID NO: 1, SEQ ID NO: 2 or SEQ ID NO: 3;
(c) a Kidney Injury Novel Gene-1 polypeptide, preferably the polypeptide of SEQ ID NO: 4, SEQ ID NO: 5 or SEQ ID NO: 6 or a fragment thereof; or
(d) an antibody against a Kidney Injury Novel Gene-1 polypeptide, preferably to the polypeptide of SEQ ID NO: 4, SEQ ID NO: 5 or SEQ ID NO: 6.

17. Screening kit for identifying e.g. agonists, antagonists, ligands, receptors, substrates, enzymes for Kidney Injury Novel Gene-1 polypeptides; or compounds which decrease or enhance the production of Kidney Injury Novel Gene-1 polypeptides, which comprises:
(a) a Kidney Injury Novel Gene-1 polypeptide, preferably that of SEQ ID NO: 4, SEQ ID NO: 5 or SEQ ID NO: 6;
(b) a recombinant cell expressing a Kidney Injury Novel Gene-1 polypeptide, preferably that of SEQ ID NO: 4, SEQ ID NO: 5 or SEQ ID NO: 6;
(c) a cell membrane expressing a Kidney Injury Novel Gene-1 polypeptide; preferably that of SEQ ID NO: 4, SEQ ID NO: 5 or SEQ ID NO: 6; or
(d) an antibody against a Kidney Injury Novel Gene-1 polypeptide, preferably that of SEQ ID NO: 4, SEQ ID NO: 5 or SEQ ID NO: 6.


LISTE DE SEQUENCES

<110> SANOFI-SYNTHELABO

<120> Kidney Injury Novel Gene-1, its isolation and
      therapeutic applications

<130> KING-1

<140>
<141>

<160> 6

<170> PatentIn Ver. 2.1

<210> 1
<211> 895
<212> ADN
<213> Homo sapiens

<220>
<221> CDS
<222> (6)..(860)

<220>
<221> 5'UTR
<222> (1)..(5)

<220>
<221> 3'UTR
<222> (861)..(895)

<300>

<400> 1
ggagg atg gcg gag ttg gta cct ttt gcg gtt ccc atc gag agt gac aaa 50
      Met Ala Glu Leu Val Pro Phe Ala Val Pro Ile Glu Ser Asp Lys
        1               5                  10                  15

acc ttg cta gtg tgg gag ctg agc tcc gga ccc acg gcc gag gct ttg   98
Thr Leu Leu Val Trp Glu Leu Ser Ser Gly Pro Thr Ala Glu Ala Leu
                 20                  25                  30

cat cat tct ctg ttc aca gca ttt tct cag ttt ggc ctt ctg tat tca   146
His His Ser Leu Phe Thr Ala Phe Ser Gln Phe Gly Leu Leu Tyr Ser
             35                  40                  45

gtc cgg gtc ttc cca aat gct gca gtg gcc cat cct ggt ttc tat gcc   194
Val Arg Val Phe Pro Asn Ala Ala Val Ala His Pro Gly Phe Tyr Ala
         50                  55                  60

gtc att aag ttt tat tct gca agg gct gcc cac aga gcc caa aag gca   242
Val Ile Lys Phe Tyr Ser Ala Arg Ala Ala His Arg Ala Gln Lys Ala
     65                  70                  75

tgc gac cgg aaa cag ctt ttt cag aaa tct cca gtc aag gtt cgt ctt   290
Cys Asp Arg Lys Gln Leu Phe Gln Lys Ser Pro Val Lys Val Arg Leu
 80                  85                  90                  95

ggc acc aga cat aag gca gtt caa cat caa gcc ctt gcc ctg aac agt   338
Gly Thr Arg His Lys Ala Val Gln His Gln Ala Leu Ala Leu Asn Ser
                100                 105                 110

tcc aaa tgc caa gaa ctg gcg aat tac tac ttt ggt ttc aat ggg tgt 386
Ser Lys Cys Gln Glu Leu Ala Asn Tyr Tyr Phe Gly Phe Asn Gly Cys
            115                 120                 125

tcc aaa agg atc atc aag ctt cag gag ctt tct gac ctt gaa gaa agg 434
Ser Lys Arg Ile Ile Lys Leu Gln Glu Leu Ser Asp Leu Glu Glu Arg
        130                 135                 140

gaa aat gaa gat agc atg gtg cca ctt ccg aag caa agc ctg aag ttc 482
Glu Asn Glu Asp Ser Met Val Pro Leu Pro Lys Gln Ser Leu Lys Phe
    145                 150                 155

ttc tgt gct tta gaa gtg gtg ttg cca tcc tgt gat tgc agg agt cct 530
Phe Cys Ala Leu Glu Val Val Leu Pro Ser Cys Asp Cys Arg Ser Pro
160                 165                 170                 175

ggc att ggc ttg gtg gag gag cct atg gat aag gtg gag gaa gga cca 578
Gly Ile Gly Leu Val Glu Glu Pro Met Asp Lys Val Glu Glu Gly Pro
                180                 185                 190

tta tca ttc ctt atg aaa agg aag aca gcc cag aag ctt gct att cag 626
Leu Ser Phe Leu Met Lys Arg Lys Thr Ala Gln Lys Leu Ala Ile Gln
            195                 200                 205

aag gct ttg tca gat gca ttc cag aaa ctg ctg att gtt gtt cta gaa 674
Lys Ala Leu Ser Asp Ala Phe Gln Lys Leu Leu Ile Val Val Leu Glu
        210                 215                 220

agt ggt aaa ata gct gtg gag tac aga ccc agt gaa gac atc gta ggt 722
Ser Gly Lys Ile Ala Val Glu Tyr Arg Pro Ser Glu Asp Ile Val Gly
    225                 230                 235

gtc aga tgc gaa gaa gaa cta cac ggt tta att caa gtc cct tgc tct 770
Val Arg Cys Glu Glu Glu Leu His Gly Leu Ile Gln Val Pro Cys Ser
240                 245                 250                 255

ccc tgg aag cag tat ggc caa gag gag gaa ggg tat ctc tcg gat ttc 818
Pro Trp Lys Gln Tyr Gly Gln Glu Glu Glu Gly Tyr Leu Ser Asp Phe
                260                 265                 270

agc ttg gag gag gaa gag ttc agg ctg cca gaa ctt gac tag 860
Ser Leu Glu Glu Glu Glu Phe Arg Leu Pro Glu Leu Asp
275 280 285

catttctgaa tatcccaagg cgaggtcatg taact 895

<210> 2
<211> 284
<212> PRT
<213> Homo sapiens

<400> 2
Met Ala Glu Leu Val Pro Phe Ala Val Pro Ile Glu Ser Asp Lys Thr
1               5                   10                  15
Leu Leu Val Trp Glu Leu Ser Ser Gly Pro Thr Ala Glu Ala Leu His
            20                  25                  30
His Ser Leu Phe Thr Ala Phe Ser Gln Phe Gly Leu Leu Tyr Ser Val
        35                  40                  45
Arg Val Phe Pro Asn Ala Ala Val Ala His Pro Gly Phe Tyr Ala Val
    50                  55                  60
Ile Lys Phe Tyr Ser Ala Arg Ala Ala His Arg Ala Gln Lys Ala Cys
65                  70                  75                  80
Asp Arg Lys Gln Leu Phe Gln Lys Ser Pro Val Lys Val Arg Leu Gly

                  85                  90                  95
Thr Arg His Lys Ala Val Gln His Gln Ala Leu Ala Leu Asn Ser Ser
            100                 105                 110
Lys Cys Gln Glu Leu Ala Asn Tyr Tyr Phe Gly Phe Asn Gly Cys Ser
        115                 120                 125
Lys Arg Ile Ile Lys Leu Gln Glu Leu Ser Asp Leu Glu Glu Arg Glu
    130                 135                 140
Asn Glu Asp Ser Met Val Pro Leu Pro Lys Gln Ser Leu Lys Phe Phe
145                 150                 155                 160
Cys Ala Leu Glu Val Val Leu Pro Ser Cys Asp Cys Arg Ser Pro Gly
                165                 170                 175
Ile Gly Leu Val Glu Glu Pro Met Asp Lys Val Glu Glu Gly Pro Leu
            180                 185                 190
Ser Phe Leu Met Lys Arg Lys Thr Ala Gln Lys Leu Ala Ile Gln Lys
        195                 200                 205
Ala Leu Ser Asp Ala Phe Gln Lys Leu Leu Ile Val Val Leu Glu Ser
    210                 215                 220
Gly Lys Ile Ala Val Glu Tyr Arg Pro Ser Glu Asp Ile Val Gly Val
225                 230                 235                 240
Arg Cys Glu Glu Glu Leu His Gly Leu Ile Gln Val Pro Cys Ser Pro
                245                 250                 255
Trp Lys Gln Tyr Gly Gln Glu Glu Glu Gly Tyr Leu Ser Asp Phe Ser
            260                 265                 270
Leu Glu Glu Glu Glu Phe Arg Leu Pro Glu Leu Asp
        275                 280

<210> 3
<211> 933
<212> ADN
<213> Homo sapiens

<220>
<221> CDS
<222> (6)..(761)

<220>
<221> 5'UTR
<222> (1)..(5)

<220>
<221> 3'UTR
<222> (762)..(933)

<400> 3
ggagg atg gcg gag ttg gta cct ttt gcg gtt ccc atc gag agt gac aaa 50
      Met Ala Glu Leu Val Pro Phe Ala Val Pro Ile Glu Ser Asp Lys
      1               5                   10                  15

acc ttg cta gtg tgg gag ctg agc tcc gga ccc acg gcc gag gct ttg    98
Thr Leu Leu Val Trp Glu Leu Ser Ser Gly Pro Thr Ala Glu Ala Leu
                20                  25                  30

cat cat tct ctg ttc aca gca ttt tct cag ttt ggc ctt ctg tat tca    146
His His Ser Leu Phe Thr Ala Phe Ser Gln Phe Gly Leu Leu Tyr Ser
            35                  40                  45

gtc cgg gtc ttc cca aat gct gca gtg gcc cat cct ggt ttc tat gcc    194
Val Arg Val Phe Pro Asn Ala Ala Val Ala His Pro Gly Phe Tyr Ala
        50                  55                  60

gtc att aag ttt tat tct gca agg gct gcc cac aga gcc caa aag gca    242
Val Ile Lys Phe Tyr Ser Ala Arg Ala Ala His Arg Ala Gln Lys Ala

65                  70                  75

tgc gac cgg aag cag ctt ttt cag aaa tct cca gtc aag gtt cgt ctt     290
Cys Asp Arg Lys Gln Leu Phe Gln Lys Ser Pro Val Lys Val Arg Leu
 80                  85                  90                  95

ggc acc aga cat aag gca gtt caa cat caa gcc ctt gcc ctg aac agt     338
Gly Thr Arg His Lys Ala Val Gln His Gln Ala Leu Ala Leu Asn Ser
                100                 105                 110

tcc aaa tgc caa gaa ctg gcg aat tac tac ttt ggt ttc aat ggg tgt     386
Ser Lys Cys Gln Glu Leu Ala Asn Tyr Tyr Phe Gly Phe Asn Gly Cys
            115                 120                 125

tcc aaa agg atc atc aag ctt cag gag ctt tct gac ctt gaa gaa agg     434
Ser Lys Arg Ile Ile Lys Leu Gln Glu Leu Ser Asp Leu Glu Glu Arg
        130                 135                 140

gaa aat gaa gat agc atg gtg cca ctt ccg aag caa agc ctg aag ttc     482
Glu Asn Glu Asp Ser Met Val Pro Leu Pro Lys Gln Ser Leu Lys Phe
    145                 150                 155

ttc tgt gct tta gaa gtg gtg ttg cca tcc tgt gat tgc agg agt cct     530
Phe Cys Ala Leu Glu Val Val Leu Pro Ser Cys Asp Cys Arg Ser Pro
160                 165                 170                 175

ggc att ggc ttg gtg gag gag cct atg gat aag gtg gag aaa gaa agt     578
Gly Ile Gly Leu Val Glu Glu Pro Met Asp Lys Val Glu Lys Glu Ser
                180                 185                 190

ggt aaa ata gct gtg gag tac aga ccc agt gaa gac atc gta ggt gtc     626
Gly Lys Ile Ala Val Glu Tyr Arg Pro Ser Glu Asp Ile Val Gly Val
            195                 200                 205

aga tgc gaa gaa gaa cta cac ggt tta att caa gtc cct tgc tct ccc     674
Arg Cys Glu Glu Glu Leu His Gly Leu Ile Gln Val Pro Cys Ser Pro
        210                 215                 220

tgg aag cag tat ggc caa gag gag gaa ggg tat ctc tcg gat ttc agc     722
Trp Lys Gln Tyr Gly Gln Glu Glu Glu Gly Tyr Leu Ser Asp Phe Ser
    225                 230                 235

ttg gag gag gaa gag ttc agg ctg cca gaa ctt gac tag catttctgaa     771
Leu Glu Glu Glu Glu Phe Arg Leu Pro Glu Leu Asp
240                 245                 250

tatcccaagg cgaggtcatg taacttcctt gggaagctct gtcgtgcccc taccccacgc 831

cgccccacca ctataggctg ctggagtcct ggaaccacct gggtcggccg cccaaatctt 891

tcctggcgaa ggggagggaa ggggtgttct ggtggagtgg ga                    933

<210> 4
<211> 251
<212> PRT
<213> Homo sapiens

<400> 4
Met Ala Glu Leu Val Pro Phe Ala Val Pro Ile Glu Ser Asp Lys Thr
  1               5                  10                  15
Leu Leu Val Trp Glu Leu Ser Ser Gly Pro Thr Ala Glu Ala Leu His
             20                  25                  30
His Ser Leu Phe Thr Ala Phe Ser Gln Phe Gly Leu Leu Tyr Ser Val

35                  40                  45
Arg Val Phe Pro Asn Ala Ala Val Ala His Pro Gly Phe Tyr Ala Val
    50                  55                  60
Ile Lys Phe Tyr Ser Ala Arg Ala Ala His Arg Ala Gln Lys Ala Cys
65                  70                  75                  80
Asp Arg Lys Gln Leu Phe Gln Lys Ser Pro Val Lys Val Arg Leu Gly
                85                  90                  95
Thr Arg His Lys Ala Val Gln His Gln Ala Leu Ala Leu Asn Ser Ser
            100                 105                 110
Lys Cys Gln Glu Leu Ala Asn Tyr Tyr Phe Gly Phe Asn Gly Cys Ser
        115                 120                 125
Lys Arg Ile Ile Lys Leu Gln Glu Leu Ser Asp Leu Glu Glu Arg Glu
    130                 135                 140
Asn Glu Asp Ser Met Val Pro Leu Pro Lys Gln Ser Leu Lys Phe Phe
145                 150                 155                 160
Cys Ala Leu Glu Val Val Leu Pro Ser Cys Asp Cys Arg Ser Pro Gly
                165                 170                 175
Ile Gly Leu Val Glu Glu Pro Met Asp Lys Val Glu Lys Glu Ser Gly
            180                 185                 190
Lys Ile Ala Val Glu Tyr Arg Pro Ser Glu Asp Ile Val Gly Val Arg
        195                 200                 205
Cys Glu Glu Glu Leu His Gly Leu Ile Gln Val Pro Cys Ser Pro Trp
    210                 215                 220
Lys Gln Tyr Gly Gln Glu Glu Glu Gly Tyr Leu Ser Asp Phe Ser Leu
225                 230                 235                 240
Glu Glu Glu Glu Phe Arg Leu Pro Glu Leu Asp
                245                 250

<210> 5
<211> 1108
<212> ADN
<213> Rattus norvegicus

<220>
<221> CDS
<222> (12)..(857)

<220>
<221> 5'UTR
<222> (1)..(11)

<220>
<221> 3'UTR
<222> (858)..(1108)

<220>
<221> polyA_signal
<222> (1072)..(1077)

<220>
<221> polyA_site
<222> (1089)..(1108)

<400> 5
gtgattttac c atg gcg gag tta atc tct ttc gta gtt ccc acc cag agc      50
             Met Ala Glu Leu Ile Ser Phe Val Val Pro Thr Gln Ser
             1               5                   10

gac aaa gtt ttg ttg gtg tgg gag ttg agc gct ggt ccc cca gcc gag      98
Asp Lys Val Leu Leu Val Trp Glu Leu Ser Ala Gly Pro Pro Ala Glu
    15                  20                  25

gcc tta agt cat tct ctg ttc aca gtg ttc tcc cag ttt ggc ctt ctg     146
Ala Leu Ser His Ser Leu Phe Thr Val Phe Ser Gln Phe Gly Leu Leu
 30                 35                  40                  45

tat tca gtc cga gtc ttc ccg aat gct gca gtg gcc ggt cct ggt ttc     194
Tyr Ser Val Arg Val Phe Pro Asn Ala Ala Val Ala Gly Pro Gly Phe
                50                  55                  60

tac gcc atc gtc aag ttt tac tcc tcg agg gac gca cag aga gcc caa     242
Tyr Ala Ile Val Lys Phe Tyr Ser Ser Arg Asp Ala Gln Arg Ala Gln
            65                  70                  75

aag gca tgc gac gga aag ccc ctt ttt cag aca tca cca gtg aag gtt     290
Lys Ala Cys Asp Gly Lys Pro Leu Phe Gln Thr Ser Pro Val Lys Val
        80                  85                  90

cgt ctt ggc acc aga cat aag gca ctg caa cat cag gct ttt gcc ttg     338
Arg Leu Gly Thr Arg His Lys Ala Leu Gln His Gln Ala Phe Ala Leu
    95                 100                 105

aat agc tca cgg tgc cag gaa ctg gca aat tac tac ttt ggc ttc aat     386
Asn Ser Ser Arg Cys Gln Glu Leu Ala Asn Tyr Tyr Phe Gly Phe Asn
110                 115                 120                 125

gga tgg tca aaa agg atc gtc aag ctg cag gag ctc tct gga ttg gag     434
Gly Trp Ser Lys Arg Ile Val Lys Leu Gln Glu Leu Ser Gly Leu Glu
                130                 135                 140

gat gca gct ctc gct gtg ccc atg cag aag cag aac ccc cag ttc ttc     482
Asp Ala Ala Leu Ala Val Pro Met Gln Lys Gln Asn Pro Gln Phe Phe
            145                 150                 155

tgt gct gta gag gtg gtg ctg ccg gcc tac ggg tgc acg agt cct gga     530
Cys Ala Val Glu Val Val Leu Pro Ala Tyr Gly Cys Thr Ser Pro Gly
        160                 165                 170

gtt ggc atc tct gag gag cct ctg cct cag ctg gag gaa ggg cag cca     578
Val Gly Ile Ser Glu Glu Pro Leu Pro Gln Leu Glu Glu Gly Gln Pro
    175                 180                 185

tcg ttt ctt acg aag agg aag aca gcg cag aag ctc gcc ttc cag gcg     626
Ser Phe Leu Thr Lys Arg Lys Thr Ala Gln Lys Leu Ala Phe Gln Ala
190                 195                 200                 205

gct tta tca gac gcc ttc cag aag ctg acc atc gtg gtt cta gaa agt     674
Ala Leu Ser Asp Ala Phe Gln Lys Leu Thr Ile Val Val Leu Glu Ser
                210                 215                 220

ggc cgg atc gcg gtg gag tac aga ccc aca gaa gag gat ctc gat gcc     722
Gly Arg Ile Ala Val Glu Tyr Arg Pro Thr Glu Glu Asp Leu Asp Ala
            225                 230                 235

aga agt gaa gaa gaa ctg cag aat tta atc cag gtg ggc tgc ttt ccc     770
Arg Ser Glu Glu Glu Leu Gln Asn Leu Ile Gln Val Gly Cys Phe Pro
        240                 245                 250

tcc agt cag tcc agc caa agg gaa gaa gaa tgt ctc tcg gac ttc agc     818
Ser Ser Gln Ser Ser Gln Arg Glu Glu Glu Cys Leu Ser Asp Phe Ser
    255                 260                 265

ctg gag gag gaa gac ctg aag ctg tgt gga ctg cac tag ccgctctgac     867
Leu Glu Glu Glu Asp Leu Lys Leu Cys Gly Leu His
270                 275                 280

tgtgccggga cctgccattt gactctgaca gatgttatcc atttccagtc agtgggttgc 927

tggcatccct gaagacccaa gtgtgaggcc tggatcttgc taggctgtgt aggaggacgc 987

cttgggagag gtctaatgga ggcttgagcc ctgtcacttc ggggggaaaa agttccccat 1047

acagaaagga gctctgtccc acataataaa ggctgcctaa caaaaaaaaa aaaaaaaaaa 1107

a 1108

<210> 6
<211> 281
<212> PRT
<213> Rattus norvegicus

<400> 6
Met Ala Glu Leu Ile Ser Phe Val Val Pro Thr Gln Ser Asp Lys Val
1               5                   10                  15
Leu Leu Val Trp Glu Leu Ser Ala Gly Pro Pro Ala Glu Ala Leu Ser
            20                  25                  30
His Ser Leu Phe Thr Val Phe Ser Gln Phe Gly Leu Leu Tyr Ser Val
        35                  40                  45
Arg Val Phe Pro Asn Ala Ala Val Ala Gly Pro Gly Phe Tyr Ala Ile
    50                  55                  60
Val Lys Phe Tyr Ser Ser Arg Asp Ala Gln Arg Ala Gln Lys Ala Cys
65                  70                  75                  80
Asp Gly Lys Pro Leu Phe Gln Thr Ser Pro Val Lys Val Arg Leu Gly
                85                  90                  95
Thr Arg His Lys Ala Leu Gln His Gln Ala Phe Ala Leu Asn Ser Ser
            100                 105                 110
Arg Cys Gln Glu Leu Ala Asn Tyr Tyr Phe Gly Phe Asn Gly Trp Ser
        115                 120                 125
Lys Arg Ile Val Lys Leu Gln Glu Leu Ser Gly Leu Glu Asp Ala Ala
    130                 135                 140
Leu Ala Val Pro Met Gln Lys Gln Asn Pro Gln Phe Phe Cys Ala Val
145                 150                 155                 160
Glu Val Val Leu Pro Ala Tyr Gly Cys Thr Ser Pro Gly Val Gly Ile
                165                 170                 175
Ser Glu Glu Pro Leu Pro Gln Leu Glu Glu Gly Gln Pro Ser Phe Leu
            180                 185                 190
Thr Lys Arg Lys Thr Ala Gln Lys Leu Ala Phe Gln Ala Ala Leu Ser
        195                 200                 205
Asp Ala Phe Gln Lys Leu Thr Ile Val Val Leu Glu Ser Gly Arg Ile
    210                 215                 220
Ala Val Glu Tyr Arg Pro Thr Glu Glu Asp Leu Asp Ala Arg Ser Glu
225                 230                 235                 240
Glu Glu Leu Gln Asn Leu Ile Gln Val Gly Cys Phe Pro Ser Ser Gln
                245                 250                 255
Ser Ser Gln Arg Glu Glu Glu Cys Leu Ser Asp Phe Ser Leu Glu Glu
            260                 265                 270
Glu Asp Leu Lys Leu Cys Gly Leu His
        275                 280